(19) Europäisches Patentamt
European Patent Office
Office européen des brevets

(11) EP 4 785 890 A1

(12) EUROPEAN PATENT APPLICATION
published in accordance with Art. 153(4) EPC

(43) Date of publication:
05.08.2026 Bulletin 2026/32

(51) International Patent Classification (IPC):
A61B 34/10 (2016.01)     A61B 17/34 (2006.01)

(21) Application number: 25845998.1

(22) Date of filing: 25.11.2025

(86) International application number:
PCT/CN2025/137526

(87) International publication number:
WO 2026/114224 (04.06.2026 Gazette 2026/23)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 26.11.2024 CN 202411702026

(71) Applicant: Shanghai Simpletouch Robot Co., Ltd.
Shanghai 201613 (CN)

(72) Inventors:
• LIU, Jian
Shanghai 201613 (CN)
• NIU, Bing
Shanghai 201613 (CN)

(74) Representative: Altmann Stößel Dick
Patentanwälte PartG mbB
Theodor-Heuss-Anlage 2
68165 Mannheim (DE)

(54) **PUNCTURE PATH PLANNING METHOD AND PLANNING SYSTEM**

(57) The present disclosure discloses a puncture path planning method and a planning system, which, based on ideas of optics and computer rendering, rapidly calculates preferred puncture path information for providing reference to medical personnel, serving as an auxiliary means for information acquisition in clinical practice. The method includes: constructing a three-dimensional model based on medical images; defining organs and tissues as units with different light absorption degrees; assigning light absorption coefficients to each unit according to whether the biopsy needle is allowed to pass and the puncture risk level; placing a light source on a puncture target region; and obtaining grayscale information on the skin surface by analyzing light absorption along different paths, wherein the grayscale information is used to provide feedback on the recommendation level of each puncture starting point. The present disclosure transforms the selection of a puncture path into the problem of finding an optimal puncture starting point, and, by combining the principles of optical propagation, proposes a new concept for planning and recommending puncture paths, which can visually indicate the positions of preferred puncture starting points in a more comprehensive and reliable manner.

FIG. 1

**Description**

**TECHNICAL FIELD**

The present disclosure relates to the technical field of medical treatment and computers, and specifically to a puncture path planning method and a planning system.

**BACKGROUND ART**

In clinical practice, biopsy needles are often selected for sampling in order to perform biopsy operations on internal organs or tissues. However, during the use of the biopsy needle, the biopsy needle will puncture the skin, muscles, and other internal organs or tissues, which inevitably causes a certain degree of damage to the human body. It is particularly important that certain organs or tissues cannot be penetrated by the biopsy needle, such as hard bone tissues including ribs, sternum, and spine (unless the target is bone marrow aspiration); dangerous locations such as the heart, large blood vessels, and trachea; and inappropriate puncture areas (for example, when the puncture target is located in the lung, the needle should not pass through organs such as the liver).

The traditional puncture surgery relies heavily on the experience of the doctor for selecting the puncture path, and there is still significant room for improvement in the reliability of selecting the puncture path. To improve the reliability of puncture path selection, the existing improved methods propose constructing a three-dimensional model of the human body based on the medical imaging of patients. The forbidden zones and safe zones are provided within the three-dimensional model, and the optimal puncture path is automatically screened using the data processing capability of a computer and preset screening conditions. This improved method can indeed accurately confirm the positions of organs and tissues within the body for the puncture target, but there are still some shortcomings in the path planning, for example, including the following conditions.

1) The risk levels associated with the biopsy needle passing through different organs and tissues vary. Simply classifying organs and tissues as safe and forbidden puncture areas may result in path options that are not objective or accurate enough.
2) The computer directly outputs the optimal screening result based on preset conditions, lacking a comparative presentation of various paths, which fails to provide the operator with more comprehensive reference information.
3) The path planning is performed in a brute-force exhaustive approach for all positions on the skin, leading to a large amount of calculation and low computational efficiency.

**SUMMARY**

A technical objective of the present disclosure is to design a new puncture path planning method and a planning system to address the shortcomings in the prior art, thereby providing more comprehensive and reliable reference information for puncture operations in clinical practice.

To achieve the above technical objective, the present disclosure provides the following technical solutions.

A puncture path planning method includes:

acquiring medical images of a puncture target and constructing a three-dimensional model of a body of the puncture target based on the medical images;

determining a location of a puncture target region within the three-dimensional model, and providing a virtual light source at the target region, wherein the virtual light source outputs equal light intensity in all directions;

screening a candidate region of a puncture starting point on a skin surface of the three-dimensional model, and determining a light propagation path from the virtual light source to each voxel point in the candidate region;

defining organs and tissues included in the three-dimensional model as units with different light absorption degrees, and assigning light absorption coefficients to corresponding units respectively based on whether a biopsy needle is allowed to pass through and a risk level of the biopsy needle when passing through; and

calculating a light intensity reaching each voxel point in the candidate region in conjunction with the light absorption coefficients of the units through which each propagation path passes, and generating a grayscale image of the candidate region based on the light intensity, wherein a recommendation degree for each voxel point in the candidate region as the puncture starting point is described by the grayscale.

Based on the above solution, the further improved solutions are as follows.

In a preferred embodiment, a step of screening the candidate region of the puncture starting point on the skin surface of the three-dimensional model includes:

in response to the puncture target region being a target point, determining a sphere with the target point as a center and a maximum puncture depth of the biopsy needle as a radius, wherein the sphere intersects with the three-dimensional model, and the skin surface of the three-dimensional model located within the sphere is selected as the candidate region; and

in response to the puncture target region being a target polyhedron, outwardly expanding along an outer surface of the target polyhedron at equal distances, with an expansion distance being the maximum puncture depth of the biopsy needle, to obtain an enlarged polyhedron, wherein the enlarged polyhedron intersects with the three-dimensional model, and the skin surface of the three-dimensional model located within the enlarged polyhedron is selected as the candidate region.

In a preferred embodiment, when a boundary of the sphere or the enlarged polyhedron exceeds a scanning range of medical images such as CT or MR, image expansion is performed using a zero-padding operation.

In a preferred embodiment, a transmitted light intensity for each unit along the light propagation path is calculated using the following formula:

$$-log_{10}\left(\frac{I_{ti}}{I_{0i}}\right) = k_i \cdot l_i,$$

where $i$ is a unit number along the light propagation path, $I_{0i}$ is an incident light intensity at unit $i$, $I_{ti}$ is a transmitted light intensity at unit $i$, $k_i$ is a light absorption coefficient of unit $i$, and $l_i$ is a length of a current light propagation passing through the unit $i$.

In a preferred embodiment, a step of assigning each voxel included within each unit separately in response to the light absorption coefficients being assigned includes:

presetting high-risk units and setting the light absorption coefficient for each voxel of all high-risk units; and for other units through which the biopsy needle can pass excluding the high-risk units, assigning different light absorption coefficients to voxels that belong to the same unit but are located at different positions based on distances from the high-risk units, wherein an assignment principle is that the farther the distances, the smaller the light absorption coefficients of the voxels.

In a preferred embodiment, a method of calculating the light intensity reaching each voxel point in the candidate region includes:

converting voxel data of the skin surface of the three-dimensional model into a geometric mesh composed of triangular facets using the Marching Cubes algorithm, and calculating a surface normal vector ($N$) of each triangular facet using coordinates of three vertices of each triangular facet to determine an orientation of each triangular facet; for a triangular facet at any one position in the candidate region, the light intensity is expressed as:

$$I = I_t * max(N \cdot L, 0) \quad \text{or} \quad I = w * I_t * max(N \cdot L, 0),$$

where $L$ represents a direction vector from a current triangular facet to the virtual light source, and $\omega$ represents a weighting coefficient;

$$I_t = I_0 \cdot 10^{-\sum_i k_i \cdot l_i},$$

where $i$ is a unit number along the light propagation path, $I_0$ is a light intensity output by the virtual light source, $k_i$ is a light absorption coefficient of unit $i$, and $l_i$ is a length of a current light propagation path passing through the unit $i$.

In a preferred embodiment, the method provided by the present disclosure accelerates the process of volume rendering of the three-dimensional model by empty space skipping, wherein the empty volumes include fully transparent regions and fully opaque regions;

the fully transparent regions correspond to background regions of the three-dimensional model, invalid regions, or preset low-risk units where the biopsy needle passes through, wherein in response to the light propagation path reaching the fully transparent regions, skipping the calculation for the fully transparent regions; and

the fully opaque regions correspond to regions through which the biopsy needle cannot pass, wherein the light propagation path encountering the fully opaque regions, skipping the subsequent calculation, and directly outputting a light intensity at a voxel point on the skin surface of the three-dimensional model reached by a current light propagation path as 0.

In a preferred embodiment, the method provided by the present disclosure further includes a step of mapping the skin surface of the three-dimensional model onto a planar map using an equidistant cylindrical projection.

In a preferred embodiment, the method provided by the present disclosure further includes a step of recognizing and classifying organs and tissues, and/or detecting lesions using a trained neural network model.

A puncture path planning system includes the following components:

a model construction module, configured to construct a three-dimensional model of a body of a puncture target based on medical images of the puncture target;

a coefficient assignment module, configured to define organs and tissues included in the three-dimensional model as units with different light absorption degrees, and assign light absorption coefficients to the units respectively based on whether a biopsy needle is allowed to pass through and a risk level of the biopsy needle when passing through;

a virtual light source configuration module, configured to generate a virtual light source at a puncture target region based on a location of the puncture target region within the three-dimensional model;

a candidate region screening module, configured to screen a candidate region of a puncture starting point on a skin surface of the three-dimensional model based on a maximum puncture depth of the biopsy needle, to determine a light propagation path from the virtual light source to each position in the candidate region; and

a grayscale value calculation module, configured to calculate a light intensity reaching each voxel point in the candidate region in conjunction with the light absorption coefficients of the units through which each light propagation path passes, and generate a grayscale image of the candidate region based on the light intensity, wherein a recommendation degree for each voxel point in an appropriate candidate region as the puncture starting point is described by the grayscale.

Beneficial effects are as follows.

The present disclosure is implemented based on medical imaging and computer technology. It provides medical personnel with reference information regarding preferred puncture paths using a three-dimensional model rendered in a specific manner, and serves as an auxiliary means for obtaining information in clinical practice. The present disclosure transforms the selection of the puncture path into the problem of finding the optimal puncture starting point, and then, by combining the principles of optical propagation, proposes a new concept for planning puncture paths and recommending paths. The design is scientific and reasonable, enabling not only an intuitive visualization of the location of the preferred puncture starting point, but also a presentation of comparison results of all candidate puncture starting points, thereby providing clinical puncture operators with more comprehensive and reliable reference information, with the aim of further improving the accuracy of puncture results. Meanwhile, the method and system of the present disclosure also improve the computation of candidate paths, reduce the computational difficulty for organs or tissues such as blood vessels, and allow acceleration through existing ray-tracing computation libraries or hardware, thereby reducing the computational load and improving the output efficiency of the computer.

**BRIEF DESCRIPTION OF DRAWINGS**

FIG. 1 is a schematic diagram comparing different puncture paths;
FIG. 2 is a schematic diagram illustrating the operation of screening candidate regions;
FIG. 3 is a schematic diagram of the expansion operation;
FIG. 4 is a rendering effect diagram of a three-dimensional model;
FIG. 5 is another rendering effect diagram of a three-dimensional model; and
FIG. 6 is another rendering effect diagram of a three-dimensional model.

**DETAILED DESCRIPTION OF EMBODIMENTS**

To illustrate the design solution and technical principles of the present disclosure, the present disclosure is described in more detail below in conjunction with the drawings and specific embodiments.

Embodiment 1

Regarding the selection of the puncture path, the currently prevailing solutions are mainly divided into two stages: the first stage involves screening feasible paths, and the second stage involves selecting the optimal path.

In the first stage, the constraints used for screening paths are generally divided into hard constraints and soft constraints. The hard constraints include, for example, defining regions that cannot be passed, and setting the angle between the puncture path and the skin to be no less than a threshold. The soft constraints include, for example, planning paths to be as far as possible from critical organs and tissues that should not be contacted, and minimizing the percutaneous puncture length of the planned path. The soft constraints are preferential conditions that need to be considered during puncture path planning. The soft constraints help optimize the puncture path to improve the safety and effectiveness of the treatment. Different surgeries can be designed according to specific circumstances.

In the prior art, during the first-stage screening, the candidate path plans are generally evaluated in a brute-force exhaustive approach to determine whether these candidate paths violate the hard constraints. The main drawbacks of this approach are the large computational load and slow output. Reducing the computational load by down-sampling or decreasing the number of candidate points also reduces the resolution and the accuracy of the screening results. Calculating the distance between the puncture paths and risky organs or tissues is relatively cumbersome. For example, it is preferable for the biopsy needle to be as far away as possible from risk areas such as ribs, sternum, spine, heart, and large blood vessels. Therefore, it requires calculating the distances between all paths and all risk areas. The regions such as small blood vessels, which can be passed through but are at higher risk, and are difficult to handle.

In the prior art, the second stage usually adopts the following two approaches:

1) assigning different weights to soft constraints, calculating a score, and screening a path with an optimal score; or
2) screening by using a multi-objective optimization algorithm, such as Pareto optimization.

Since it is generally difficult to determine whether the weights assigned to the soft constraints are appropriate, multi-objective optimization methods are more commonly used.

Different from the prior art, the present disclosure proposes a new puncture path planning method, in which optical concepts are incorporated into puncture path planning.

The main implementation process of the method of the present disclosure includes the following steps:

S1: acquiring medical images of a puncture target and constructing a three-dimensional model of a body of the puncture target based on the medical images;
S2: determining a location of a puncture target region within the three-dimensional model, and providing a virtual light source at the target region, wherein the virtual light source outputs equal light intensity in all directions;
S3: screening a candidate region of a puncture starting point on a skin surface of the three-dimensional model, and determining a light propagation path from the virtual light source to each voxel point in the candidate region;
S4: defining organs and tissues included in the three-dimensional model as units with different light absorption degrees, and assigning light absorption coefficients to each unit respectively based on whether a biopsy needle is allowed to pass through and a risk level of the biopsy needle when passing through; and
S5: calculating a light intensity reaching each voxel point in the candidate region in conjunction with the light absorption coefficients of the units through which each light propagation path passes, and generating a grayscale image of the candidate region based on the light intensity, wherein a recommendation degree for each voxel point in the candidate region as the puncture starting point is described by the grayscale.

In the above process:

The medical images in the step S1 can be obtained from CT scans. Constructing the three-dimensional model based on CT images requires intermediate steps of performing detection and recognition of organs and tissues, and this step is generally implemented through segmentation using a trained neural network. In theory, a computer vision (CV) method is also applicable, but the difficulty is relatively high. When the CV method is adopted, HU value ranges of different organs and tissues need to be set. For example, a range of [x, y] is defined as bone, and a range of [z, w] is defined as lung, followed by refinement using morphological operations. When detection and recognition of organs and tissues are implemented through training a neural network, the implementation generally includes the following steps. 1) Manual annotation: the annotation software such as ITK-SNAP or 3D Slicer is used. Different organs and tissues in CT images are painted and marked based on medical knowledge. Different organs or tissues are overlaid by different masks according to preset coding rules, and the masks are stored as annotation data. 2) Model construction: the 2D or 3D segmentation network is constructed, such as a U-Net or a V-Net. 3) Model training: the annotation data and the CT images are jointly used for training. 4) Model usage: CT images to be analyzed are used as input, segmentation is performed using the trained model, and organ or tissue segmentation masks are output and stored as files for subsequent use. The construction of the three-dimensional model in the step S1 can be achieved using the prior art. Details are not further described herein.

Regarding the steps S2 to S4, the design concept is as follows.

A human body can be conceptualized as a three-dimensional object, and different tissues or organs are assigned different light transmission or absorption coefficients based on whether the biopsy needle is allowed to pass through, thereby forming a "light box". For the tissues or organs through which the biopsy needle is not allowed to pass, such as ribs, sternum, or non-target organs, a very high light absorption coefficient is assigned to make these tissues or organs opaque (or nearly opaque). For the tissues or organs through which the biopsy needle is allowed to pass, such as muscle, connective tissue, or the target lesion, a very low light absorption coefficient is assigned to make these tissues or organs highly transparent. For the tissues or organs through which the biopsy needle is allowed to pass but that carry a relatively high risk, such as blood vessels, an appropriate light absorption coefficient is assigned to make these tissues or organs partially transparent. A light source is then placed at the puncture target region, or the puncture target is transformed into a light source. The light emitted by the source, after being absorbed by multiple layers on the periphery, eventually shows varying degrees of illumination when observed externally. The areas with higher brightness are favorable puncture paths, and the areas with low brightness indicate that there are many obstacles blocking the path.

To provide an easily understandable demonstration, the demonstration herein uses two-dimensional graphics, but all calculations in the method of the present disclosure are actually performed in three-dimensional space.

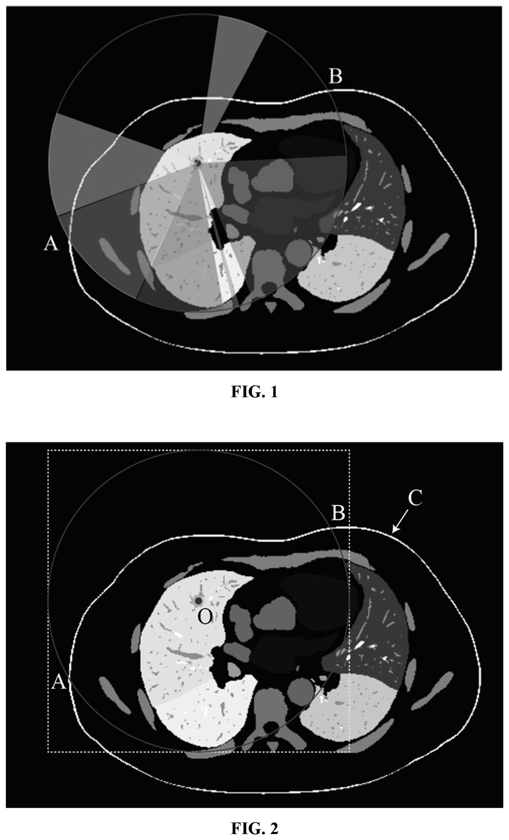

FIG. 1 shows a two-dimensional section of a selectable puncture path region, in which red points represent the puncture target points. A red circle represents a sphere drawn with the maximum puncture depth R (needle length) of the biopsy needle as the radius. When the biopsy needle cannot reach beyond the human body (insufficient length), the puncture cannot be performed. Therefore, feasible puncture paths must exist in the difference set between the sphere space and the human body space (the region from point A to point B in the figure). In the figure, yellow indicates selectable regions, blue indicates an example of a region blocked by ribs and thus impassable by the needle, orange indicates an example of a region where the path crosses a plurality of lung lobes and is thus impassable, green indicates an example of a region where the biopsy needle length is insufficient, and pink indicates an example of a region blocked by the heart or a major artery and thus impassable.

For the step S5, a transmitted light intensity for each unit along the light propagation path can be calculated using the following formula:

$$-log_{10}\left(\frac{I_{ti}}{I_{0i}}\right) = k_i \cdot l_i,$$

where $i$ is a unit number along the light propagation path, $I_{0i}$ is an incident light intensity at unit $i$, $I_{ti}$ is a transmitted light intensity at unit $i$, $k_i$ is a light absorption coefficient of unit $i$, and $l_i$ is a length of a current light propagation path passing through the unit $i$.

When a light propagation path passes through a plurality of light absorption units of organs or tissues, the light absorption of this light propagation path can be accumulated.

The above formula is a simplified formula derived based on the Lambert-Beer law. According to the Lambert-Beer law, if the incident light intensity is denoted as $I_0$, the transmitted light intensity as $I_t$, and the absorbance as $A_i$, then $A_i = -log_{10}(I_t/I_0) = K_i \cdot l_i \cdot c_i$, where $K_i$ is the absorption coefficient, $l_i$ is the length of the light passing through the target tissue or organ along the path, and $c_i$ is the molar concentration. In this model, since $c_i$ is unnecessary, the formula can be simplified. By denoting the absorption coefficient of the target tissue or organ as $k_i$, the formula shown above is obtained.

Introducing this formula can simplify the calculation process. Since this formula allows infinite subdivision for the same unit $i$ without changing the calculation result, this formula is valid even for a single voxel. Thus, the direct calculation can be performed on the voxel-level three-dimensional array, which eliminates the need to solve complex questions such as "what is the diameter of a blood vessel along the path". The problem of calculating the blood vessel diameter is transformed into the accumulated absorption effect of semi-transparent objects along the light path.

In the step S4, when the light absorption coefficients are assigned, if the distance from the biopsy needle to risky organs or tissues is also included in the constraints, each voxel contained within each unit can be assigned separately. That is, the absorption coefficients of different positions within the same unit may vary. The specific operations include:

1) presetting high-risk units and assigning the light absorption coefficient to each voxel of all high-risk units; and
2) for other units through which the biopsy needle can pass excluding the high-risk units, assigning different light absorption coefficients to voxels that belong to the same unit but are located at different positions based on the distances from the high-risk units. The assignment principle is that the farther the distances from the high-risk units, the smaller the light absorption coefficients of the voxels.

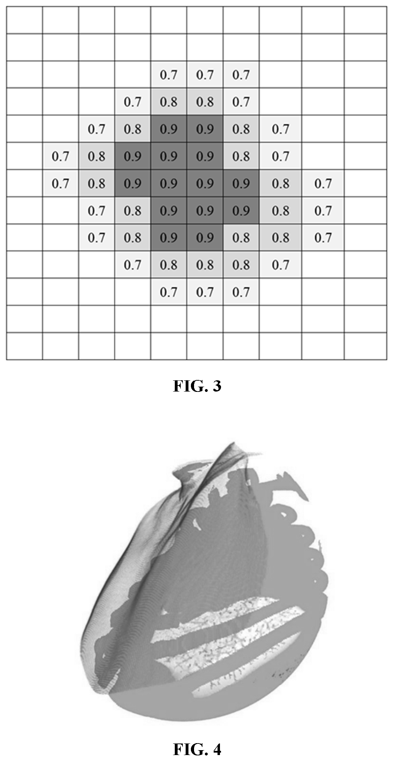

The issue of the distance between the biopsy needle and the risky organs or tissues can be addressed using a gradient expansion operation. In a practical implementation, a gradient expansion operation can be performed on the absorption coefficient array of organs or tissues using a method based on distance transform and a mapping function. As shown in FIG. 3, the farther the distance, the smaller the absorbance, so that the attenuation is greater when the light propagation path is closer to the risky organs or tissues. Distance transform is an operation used in image processing (including 2D images and 3D voxel images) to calculate a shortest distance from each voxel (or pixel) in the image to a specific set, such as a target object or the boundary of a shape. In a voxel model, for a given shape (such as a voxel set of a polyhedral shape), the distance transform calculates, for each voxel, a shortest distance from this voxel to a boundary of the shape. This distance can be a Euclidean distance (in a three-dimensional space, a Euclidean distance from voxel ($x,y$, $z$) to boundary voxel ($x_0,y_0,z_0$) is $\sqrt{(x-x_0)^2+(y-y_0)^2+(z-z_0)^2}$ ), or can be another distance metric, such as a Manhattan distance (the Manhattan distance in a three-dimensional space is $|x - x_0| + |y - y_0| + |z - z_0|$). After an image (or voxel model) is obtained through the distance transform, a mapping function can be used to implement gradient expansion attribute assignment. The voxel model after the distance transform can be stored in an array, and then a linearly decreasing attribute assignment is implemented (for example, a voxel having a distance of 0 is assigned a maximum value $V_{max}$, and as the distance increases, the assigned value decreases with a fixed step size $s$). Assuming that an attribute value after gradient expansion is $E$, a maximum attribute value is $V_{max}$, a distance is $d$, and a decreasing step size is $s$, a corresponding relationship is represented by the formula $E = max\{V_{max} - sd, 0\}$. Different mapping functions (such as an exponential function or a logarithmic function) can be designed and implemented according to specific application requirements.

In the step S5, when considering calculating the angle between the skin and the puncture path and implementing the constraint operation, a reflection model can be introduced. Since the skin has a relatively large area, calculating angles formed by lines connecting all points on the skin surface with the puncture target and the skin results in a relatively large computational burden. In addition, since the angle and the length, whether it can be passed through, the light intensity, and other variables have different dimensions, it becomes challenging to handle them simultaneously when multiple factors are considered. Therefore, a simplified reflective illumination model is introduced to address this issue.

In the model of the present disclosure, diffraction and polarization of light are not considered, scattering is not involved, and reflection and refraction of light rays are calculated at most once. A principle of a Lambert illumination model is referenced to simplify the calculation. The Lambert illumination model is a commonly used diffuse reflection illumination model, which describes an interaction between a surface of an object and a light source. Assuming that the surface is a perfectly diffuse surface, an illumination intensity is proportional to a cosine value of an angle between a light direction and a surface normal vector. Accordingly, formula $I = I_{light} \cdot max(0, \mathbf{N} \cdot \mathbf{L})$ is obtained, where $I$ represents a final illumination intensity, $I_{light}$ represents an intensity of a light source, $\mathbf{N}$ represents a surface normal vector, $\mathbf{L}$ represents a direction vector from a surface point to the light source, and $\mathbf{N} \cdot \mathbf{L}$ represents a dot product between the normal vector and the light source direction, namely a cosine value of the angle. The calculation of the surface normal vector constitutes a core of the Lambert illumination model, and a Marching Cubes technique is capable of providing a normal vector for each surface point, thereby supporting Lambert illumination calculation. The Marching Cubes algorithm is an algorithm for extracting an isosurface from three-dimensional volume data, and a result of the algorithm is generation of a group of surface meshes composed of triangular facets. The surface meshes define a surface of an object in the volume data. Importantly, the Marching Cubes algorithm not only generates the surface, but also provides vertex information of each triangular facet, such that a normal vector of each vertex is further calculated. Regarding the surface meshes, an output of the Marching Cubes algorithm is a group of triangular facets, and the triangular facets collectively form an isosurface in 3D volume data. The normal vector is critical in light intensity calculation, since the surface normal vector determines an interaction between light rays and a surface. A surface normal vector of each triangular facet can be calculated based on vertex positions of the triangular facet.

When calculating light intensity on a skin surface using the Marching Cubes technique, extracting a surface mesh of the skin from 3D volume data is first performed. The Marching Cubes algorithm converts voxel data into a geometric mesh composed of triangular facets, and each triangular facet is composed of three vertices $\mathbf{v_0}$, $\mathbf{v_1}$, and $\mathbf{v_2}$. Based on the coordinates of the three vertices, a surface normal vector $\mathbf{N}$ of the triangular facet is calculated according to the following expression:

$$\mathbf{N} = \frac{(\mathbf{v_1} - \mathbf{v_0}) \times (\mathbf{v_2} - \mathbf{v_0})}{\|\mathbf{N}\|} .$$

The normal vector N represents an orientation of the skin surface at the triangular facet. Therefore, by generating the surface meshes and normal vectors of the skin using the Marching Cubes technique, and further combining the Lambert illumination model, the light intensity at each position on the skin surface is accurately calculated.

To accelerate illumination calculation, a normal vector caching optimization strategy is adopted, in which the normal vectors are calculated in advance and cached during the calculation process, so as to avoid repeated calculation.

In summary, in the step S5 of the present disclosure, a method for calculating the light intensity reaching each voxel point in the candidate region is described as follows.

It converts voxel data of the skin surface of the three-dimensional model into a geometric mesh composed of triangular facets using the Marching Cubes algorithm, and calculates a surface normal vector ($N$) of each triangular facet using coordinates of three vertices of each triangular facet to determine an orientation of each triangular facet.

For a triangular facet at any one position in the candidate region, the light intensity is expressed as:

$$I = I_t * max(N \cdot L, 0) \text{ or } I = w * I_t * max(N \cdot L, 0),$$

where $L$ represents a direction vector from a current triangular facet to the virtual light source, and $\omega$ represents a weighting coefficient; and

$$I_t = I_0 \cdot 10^{-\sum_i k_i \cdot l_i},$$

where $i$ is a unit number along the light propagation path, $I_0$ is a light intensity output by the virtual light source, $k_i$ is a light absorption coefficient of unit $i$, and $l_i$ is a length of a current light propagation path passing through the unit $i$.

In the above formula, the introduction of the weighting coefficient $\omega$ is mainly based on considerations of parameter adjustment and optimization. Regarding the influence of the angle between the skin and the biopsy needle, if we consider that in some cases a larger angle between the biopsy needle and the skin normal may also be acceptable, it is not necessarily worse than a smaller angle, so we can introduce a weighting coefficient into the formula. Then, a balance between $I_t$ and $I$ is adjusted by adjusting a value of $\omega$.

The setting of parameters including the light absorption coefficient, the decreasing step size, and the weighting coefficient can be adjusted manually based on clinical data testing in combination with clinical experience. The optimal puncture paths can also be manually selected and annotated, and then the parameter adjustment is performed using a machine learning method or a deep learning method.

A specific example is as follows.

1. Organ and tissue segmentation and modeling

Organ or tissue segmentation masks of a lung are first obtained using a neural network method or a machine vision and image morphology method. For example, a trained neural network is used to segment organs or tissues including lung lobes, a heart, mediastinal vessels, ribs, a sternum, a liver, a spleen, kidneys, a pancreas, a trachea, and skin.

2. Lesion segmentation and modeling

A lesion coordinate is obtained by segmentation or detection using a trained neural network, or a lesion coordinate is manually specified.

3. Data preparation

According to the modeling requirements, the masks of the above organs and tissues are assigned different numerical values to prepare the following arrays.

| Name | Operation | Example |
|---|---|---|
| Mask | It assigns values according to whether the biopsy needle is allowed to pass through an organ or tissue, so as to simplify calculation by eliminating numerical differences between similar organs or tissues. | A background is assigned as 0, a passable region is assigned as 1 (a target lung lobe, skin, muscle, and the like), a region that is passable but has risk is assigned as 2 (a pulmonary vein and a pulmonary artery), and a non-passable region is assigned as 3 (a heart, bone, and the like). |

(continued)

| Name | Operation | Example |
|---|---|---|
| Light absorption parameter (opacity) | It assigns values according to whether the biopsy needle is allowed to pass through an organ or tissue and a risk level. Taking lung puncture as an example, a target lesion is assumed to be located in a left upper lung lobe. For non-puncturable regions including a heart, mediastinal vessels, ribs, a sternum, a liver, a spleen, kidneys, a pancreas, and a trachea, relatively large light absorption values are assigned. For non-target lung lobes including a left lower lung lobe, a right upper lung lobe, a right middle lung lobe, and a right lower lung lobe, relatively large light absorption values are assigned. The relatively large light transmittance is assigned to skin and lesions (including pulmonary nodules, lung lobes, and lung cancer). The voxel values are manually assigned markers and are independent of HU values of organs or tissues. | A non-passable region is assigned a relatively large light absorption coefficient, for example, 0.9, or even 1 (representing a fully opaque region). The risk region is assigned a medium value $\alpha$, for example, 0.5. The value of $\alpha$ is determined based on clinical experience or experimental results. When an increased penalty for traversing blood vessels is desired, the value of $\alpha$ is increased. The purpose here is to penalize paths that pass through a greater number of blood vessels or vessels with larger diameters. A passable region is assigned a relatively small value, for example, 0.1, or even 0 (representing a fully transparent region). |

During the process of achieving a final illumination effect, the following optimization methods are further adopted to improve computational efficiency.

1) Empty space skipping

Empty space skipping is an optimization technique used to accelerate volume rendering, especially in ray casting and volume ray casting. When applied in the method of the present disclosure, the empty space includes fully transparent regions and fully opaque regions.

The fully transparent regions correspond to background regions of the three-dimensional model, invalid regions, or preset low-risk units where the biopsy needle passes through (for example, connective tissue, with an opacity set to 0), and when the light propagation path reaches the fully transparent regions, the calculation for the fully transparent regions is skipped.

The fully opaque regions correspond to regions through which the biopsy needle cannot pass (for example, ribs, sternum, heart, with an opacity set to 1). When a light propagation path encounters the fully opaque regions, subsequent calculation is skipped, and the light intensity of the corresponding voxel point on the skin surface of the three-dimensional model reached by the current light propagation path is directly output as 0.

2) GPU acceleration

GPU acceleration is a technique for improving computational speed and efficiency by utilizing a graphics processing unit (GPU). The GPU is specifically designed for parallel processing and contains a large number of computational cores, making it suitable for handling large-scale parallel tasks. In fields such as computer graphics, deep learning, and scientific computing, GPU acceleration plays a crucial role. Unlike a traditional CPU, this GPU has a large number of stream processors capable of processing multiple threads simultaneously. For applications such as volume rendering that require processing massive voxels and voxel data, the parallel architecture of the GPU enables execution of millions of computational operations in a short time. The basic principle of GPU-based acceleration is to divide a large-scale computational task into smaller blocks and execute these small blocks in parallel. The GPU architecture is particularly suitable for matrix operations and geometric transformations, which perform efficiently in graphics rendering and deep learning. During the process of ray casting and volume rendering, parallel computation by GPU can be used to calculate interactions between each light ray and voxel data, thereby rapidly generating images.

3) Mapping the skin surface of the three-dimensional model onto a planar map

To facilitate the observation of 3D results, the 3D skin surface can be mapped onto a planar map using a projection method. A grayscale value of each point on the planar map represents a light intensity value, and coordinates of each point can be mapped back to 3D space, thereby enabling a reversible conversion from the 2D planar map to 3D space and providing convenience for further analysis and processing. An equidistant cylindrical projection can be used, but not limited thereto. The principle and calculation of the projection are as follows. An equidistant cylindrical projection is a method for projecting an Earth surface onto a cylindrical surface and then unfolding the cylindrical surface into a plane. In this projection, meridians are projected as equally spaced vertical lines, and parallels are projected as horizontal lines. Regarding the calculation formula, assuming the target is a sphere with a radius of $R$, for a point $(\theta, \varphi)$ on the sphere (where $\theta$ is the longitude and $\varphi$ is the latitude), a corresponding point $(x, y)$ on the equidistant cylindrical projection plane can be calculated using the following formulas: $x = R\theta$, $y = R \sin \varphi$.

In principle, the equidistant cylindrical projection preserves a perpendicular relationship between meridians and parallels, and maintains equal spacing along the meridian direction. This projection method preserves relative accuracy of shapes and directions to a certain extent, enabling intuitive observation and analysis of geographic information or other three-dimensional data results on the projected plane. Meanwhile, since the coordinates of each point can be mapped back from the plane to 3D space using inverse formulas, convenience is provided for further analysis and processing.

4) ROI extraction

ROI (region of interest) refers to a specific region in an image or data that requires focused attention and detailed analysis. The introduction of an ROI is intended to concentrate on the study object, reduce interference from irrelevant information, and improve analysis efficiency and accuracy.

Based on the above concept, in the step S3 of the method, the candidate regions are selected as follows.

When the puncture target region is a target point, a sphere is determined with the target point as the center and the maximum puncture depth of the biopsy needle as the radius. The sphere intersects with the three-dimensional model, and the skin surface of the three-dimensional model within the sphere serves as the candidate region. As shown in FIG. 2, A and B in the figure indicate intersection points between the sphere and the skin, C indicates the skin, and O indicates the target point.

When the puncture target region is a target polyhedron, the outer surface of the target polyhedron is expanded outward at equal distance, with an expansion distance being the maximum puncture depth of the biopsy needle, to obtain an enlarged polyhedron, wherein the enlarged polyhedron intersects with the three-dimensional model, and the skin surface of the three-dimensional model located within the enlarged polyhedron is selected as the candidate region.

The mask and the light absorption coefficient (transparency) array are cropped along the bounding box enclosing the candidate region.

When the sphere, the expanded polyhedron, or the bounding box exceeds the scanning range of corresponding medical images, such as CT, a zero-padding operation is performed to extend the image.

The method has the following advantages:

(1) by cropping, the size of arrays is reduced, thereby decreasing computational load; and
(2) by performing a symmetrical processing on the ROI, the problem of asymmetric calculation caused by the lesion not being at the center point of the array is avoided. (If it is asymmetric, issues related to different distances in different directions and different angle value ranges need to be considered, which will increase the computational complexity.)

It should also be noted that, for the same puncture target region, each voxel point in the candidate region of the starting point corresponds to only one light propagation path. When the puncture target region is a polyhedron, only the shortest path from each candidate voxel point to the puncture target region is considered as the light propagation path in this method.

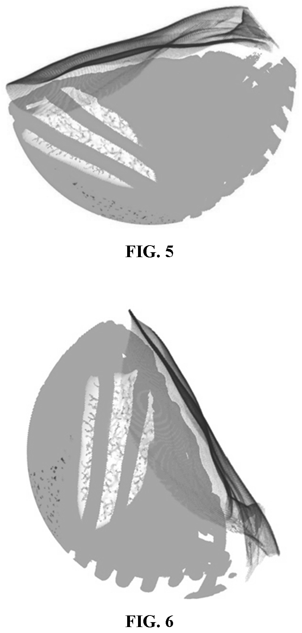

The rendering model examples are shown in FIGS. 4-6.

The purple regions represent skin, and puncture is allowed only where skin exists; the green regions represent obstacles; the red regions represent pulmonary veins and pulmonary arteries, with darker red indicating higher puncture risk; the yellow regions represent pulmonary nodules, which are the puncture targets; and the white regions represent safe areas.

Based on the foregoing method and principles, after calculating the light intensity distribution of the candidate region, the light intensity is observed. The areas with higher light intensity (higher grayscale values) indicate higher recommendation degrees for puncture starting points, and darker areas are not recommended puncture starting points. The non-candidate regions on the model surface can be directly represented as black areas. If the perspective projection is applied, the distribution can be observed on a two-dimensional plane.

This method is intuitive and easy to understand, providing more comprehensive reference information for clinical puncture operations. Compared with prior art, it can reduce computational difficulty for organs and tissues such as blood vessels and can utilize existing ray-tracing libraries or hardware for acceleration, significantly lowering computational load and improving computational efficiency.

Furthermore, this method is more suitable for planning puncture paths using rigid biopsy needles.

Embodiment 2:

A puncture path planning system includes the following components:

a model construction module, configured to construct a three-dimensional model of a body of a puncture target based on medical images of the puncture target;

a virtual light source configuration module, configured to generate a virtual light source at a puncture target region based on a location of the puncture target region within the three-dimensional model;

a candidate region screening module, configured to screen a candidate region of a puncture starting point on a skin surface of the three-dimensional model based on a maximum puncture depth of the biopsy needle, to determine a light propagation path from the virtual light source to each voxel point in the candidate region;

a coefficient assignment module, configured to define organs and tissues included in the three-dimensional model as units with different light absorption degrees, and assign light absorption coefficients to the units respectively based on whether a biopsy needle is allowed to pass through and a risk level of the biopsy needle when passing through; and

a grayscale value calculation module, configured to calculate a light intensity reaching each voxel point in the candidate region in conjunction with the light absorption coefficients of the units through which each light propagation path passes, and generate a grayscale image of the candidate region based on the light intensity, wherein a recommendation degree for each voxel point in the candidate region as the puncture starting point is described by the grayscale.

This embodiment adopts the same design concept as Embodiment 1, and the descriptions of the method and principles of this embodiment can be referred to Embodiment 1.

The above embodiments of the present disclosure have been described in conjunction with the drawings, but the present disclosure is not limited to the specific embodiments described above. The above specific embodiments are merely illustrative, not restrictive. Those skilled in the art, based on the teachings of the present disclosure, can make various modifications, changes, substitutions, and variations of these embodiments without departing from the spirit of the present disclosure and the scope protected by the claims. All such modifications, changes, substitutions, and variations fall within the protection scope of the present disclosure.

**Claims**

1. A puncture path planning method, comprising:

acquiring medical images of a puncture target and constructing a three-dimensional model of a body of the puncture target based on the medical images;

determining a location of a puncture target region within the three-dimensional model, and providing a virtual light source at the target region, wherein the virtual light source outputs equal light intensity in all directions;

screening a candidate region of a puncture starting point on a skin surface of the three-dimensional model, and determining a light propagation path from the virtual light source to each voxel point in the candidate region;

defining organs and tissues comprised in the three-dimensional model as units with different light absorption degrees, and assigning light absorption coefficients to corresponding units respectively based on whether a biopsy needle is allowed to pass through and a risk level of the biopsy needle when passing through; and

calculating a light intensity reaching each voxel point in the candidate region in conjunction with the light absorption coefficients of the units through which each light propagation path passes, and generating a grayscale image of the candidate region based on the light intensity, wherein a recommendation degree for each voxel point in the candidate region as the puncture starting point is described by the grayscale.

2. The puncture path planning method according to claim 1, wherein a step of screening a candidate region of a puncture starting point on a skin surface of the three-dimensional model comprises:

in response to the puncture target region being a target point, determining a sphere with the target point as a center and a maximum puncture depth of the biopsy needle as a radius, wherein the sphere intersects with the three-dimensional model, and the skin surface of the three-dimensional model located within the sphere is selected as the candidate region; and
in response to the puncture target region being a target polyhedron, outwardly expanding along an outer surface of the target polyhedron at equal distances, with an expansion distance being the maximum puncture depth of the biopsy needle, to obtain an enlarged polyhedron, wherein the enlarged polyhedron intersects with the three-dimensional model, and the skin surface of the three-dimensional model located within the enlarged polyhedron is selected as the candidate region.

3. The puncture path planning method according to claim 1, wherein when a boundary of the sphere or the enlarged polyhedron exceeds a scanning range of the medical images, image expansion is performed using a zero-padding operation.

4. The puncture path planning method according to claim 1, wherein a transmitted light intensity for each unit along the light propagation path is calculated using a following formula:

$$-log_{10}\left(\frac{I_{ti}}{I_{0i}}\right) = k_i \cdot l_i,$$

where $i$ is a unit number along the light propagation path, $I_{0i}$ is an incident light intensity at unit $i$, $I_{ti}$ is a transmitted light intensity at unit $i$, $k_i$ is a light absorption coefficient of unit $i$, and $l_i$ is a length of a current light propagation passing through the unit $i$.

5. The puncture path planning method according to claim 1, wherein a step of assigning each voxel included within each unit separately in response to the light absorption coefficients being assigned comprises:

presetting high-risk units and setting the light absorption coefficient for each voxel of all high-risk units; and
for other units through which the biopsy needle can pass excluding the high-risk units, assigning the different light absorption coefficients to voxels that belong to the same unit but are located at different positions based on distances from the high-risk units, wherein an assignment principle is that the farther the distances, the smaller the light absorption coefficients of the voxels.

6. The puncture path planning method according to claim 1, wherein a method of calculating the light intensity reaching each voxel point in the candidate region comprises:

converting voxel data of the skin surface of the three-dimensional model into a geometric mesh composed of triangular facets using a Marching Cubes algorithm, and calculating a surface normal vector ($N$) of each triangular facet using coordinates of three vertices of each triangular facet to determine an orientation of the each triangular facet, wherein
for a triangular facet at any one position in the candidate region, a light intensity $I$ is expressed as:

$$I = I_t * max(N \cdot L, 0) \quad \text{or} \quad I = w * I_t * max(N \cdot L, 0),$$

where $L$ represents a direction vector from a current triangular facet to the virtual light source, and $\omega$ represents a weighting coefficient; and

$$I_t = I_0 \cdot 10^{-\sum_i k_i \cdot l_i},$$

where $i$ is a unit number along the light propagation path, $I_0$ is a light intensity output by the virtual light source, $k_i$ is a light absorption coefficient of unit $i$, and $l_i$ is a length of a current light propagation path passing through unit $i$.

7. The puncture path planning method according to claim 1, comprising accelerating a process of volume rendering of the three-dimensional model by empty space skipping, wherein the empty space comprises fully transparent regions and fully opaque regions;

the fully transparent regions correspond to background regions of the three-dimensional model, invalid regions, or preset low-risk units where the biopsy needle passes through, wherein in response to the light propagation path reaching the fully transparent regions, skipping a calculation for the fully transparent regions; and
the fully opaque regions correspond to regions through which the biopsy needle cannot pass, wherein in response to the light propagation path encountering the fully opaque regions, skipping a subsequent calculation, and directly outputting a light intensity at a corresponding voxel point on the skin surface of the three-dimensional model reached by a current light propagation path as 0.

8. The puncture path planning method according to claim 1, comprising a step of mapping the skin surface of the three-dimensional model onto a planar map using an equidistant cylindrical projection.

9. The puncture path planning method according to claim 1, comprising a step of recognizing and classifying organs and tissues, and/or detecting lesions using a trained neural network model.

10. A puncture path planning system, comprising:

a model construction module, configured to construct a three-dimensional model of a body of a puncture target based on medical images of the puncture target;
a virtual light source configuration module, configured to generate a virtual light source at a puncture target region based on a location of the puncture target region within the three-dimensional model;
a candidate region screening module, configured to screen a candidate region of a puncture starting point on a skin surface of the three-dimensional model based on a maximum puncture depth of a biopsy needle, to determine a light propagation path from the virtual light source to each voxel point in the candidate region;
a coefficient assignment module, configured to define organs and tissues comprised in the three-dimensional model as units with different light absorption degrees, and assign light absorption coefficients to corresponding units respectively based on whether a biopsy needle is allowed to pass through and a risk level of the biopsy needle when passing through; and
a grayscale value calculation module, configured to calculate a light intensity reaching each voxel point in the candidate region in conjunction with the light absorption coefficients of the units through which each light propagation path passes, and generate a grayscale image of the candidate region based on the light intensity, wherein a recommendation degree for the each voxel point in the candidate region as the puncture starting point is described by the grayscale.

**FIG. 1**

**FIG. 2**

|  |  |  |  |  |  |  |  |  |  |  |
|---|---|---|---|---|---|---|---|---|---|---|
|  |  |  |  |  |  |  |  |  |  |  |
|  |  |  |  |  | 0.7 | 0.7 | 0.7 |  |  |  |
|  |  |  |  | 0.7 | 0.8 | 0.8 | 0.7 |  |  |  |
|  |  |  | 0.7 | 0.8 | 0.9 | 0.9 | 0.8 | 0.7 |  |  |
|  |  | 0.7 | 0.8 | 0.9 | 0.9 | 0.9 | 0.8 | 0.7 |  |  |
|  |  | 0.7 | 0.8 | 0.9 | 0.9 | 0.9 | 0.9 | 0.8 | 0.7 |  |
|  |  |  | 0.7 | 0.8 | 0.9 | 0.9 | 0.9 | 0.8 | 0.7 |  |
|  |  |  | 0.7 | 0.8 | 0.9 | 0.9 | 0.8 | 0.8 | 0.7 |  |
|  |  |  |  | 0.7 | 0.8 | 0.8 | 0.8 | 0.7 |  |  |
|  |  |  |  |  | 0.7 | 0.7 | 0.7 |  |  |  |
|  |  |  |  |  |  |  |  |  |  |  |
|  |  |  |  |  |  |  |  |  |  |  |

**FIG. 3**

**FIG. 4**

**FIG. 5**

**FIG. 6**

## INTERNATIONAL SEARCH REPORT

| | International application No. |
|---|---|
| | **PCT/CN2025/137526** |

### A. CLASSIFICATION OF SUBJECT MATTER

A61B34/10(2016.01)i; A61B17/34(2006.01)i

According to International Patent Classification (IPC) or to both national classification and IPC

### B. FIELDS SEARCHED

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CJFD, VCN, VEN, DWPI: 穿刺, 路径, 规划, 光源, 光强, 起始点, 穿刺点, 吸收, 风险, 皮肤, 投影, Punc+, path+, plan+, light 1d sourc+, light 1d intens+, start+ 1d point?, punct+ 1d point?, absorb+, risk+, skin, project+

### C. DOCUMENTS CONSIDERED TO BE RELEVANT

| Category* | Citation of document, with indication, where appropriate, of the relevant passages | Relevant to claim No. |
|---|---|---|
| PX | CN 119498956 A (SHANGHAI SMART TOUCH TECHNOLOGY CO., LTD.) 25 February 2025 (2025-02-25)<br>claims 1-10 | 1-10 |
| X | WO 2024032570 A1 (WUHAN UNITED IMAGING HEALTHCARE SURGICAL TECHNOLOGY CO., LTD.) 15 February 2024 (2024-02-15)<br>description, paragraphs 56-417, and figures 1-19 | 1-5, 7-10 |
| Y | WO 2024032570 A1 (WUHAN UNITED IMAGING HEALTHCARE SURGICAL TECHNOLOGY CO., LTD.) 15 February 2024 (2024-02-15)<br>description, paragraphs 56-417, and figures 1-19 | 6 |
| Y | CN 115105198 A (HANGZHOU WINNER-ONCO MEDICAL TECHNOLOGY CO., LTD.) 27 September 2022 (2022-09-27)<br>description, paragraph 45 | 6 |
| X | CN 109330667 A (TIANJIN MEDICAL UNIVERSITY CANCER INSTITUTE & HOSPITAL) 15 February 2019 (2019-02-15)<br>description, paragraphs 4-50 | 1-10 |

☑ Further documents are listed in the continuation of Box C.　　☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **12 February 2026** | **11 February 2026** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2025/137526** |

**C.      DOCUMENTS CONSIDERED TO BE RELEVANT**

| Category* | Citation of document, with indication, where appropriate, of the relevant passages | Relevant to claim No. |
|---|---|---|
| A | US 2010228534 A1 (ACTIVIEWS LTD.) 09 September 2010 (2010-09-09)<br>     entire document | 1-10 |

Form PCT/ISA/210 (second sheet) (July 2022)

**INTERNATIONAL SEARCH REPORT**
Information on patent family members

International application No.

**PCT/CN2025/137526**

| Patent document cited in search report | | | Publication date (day/month/year) | Patent family member(s) | | | Publication date (day/month/year) |
|---|---|---|---|---|---|---|---|
| CN | 119498956 | A | 25 February 2025 | None | | | |
| WO | 2024032570 | A1 | 15 February 2024 | None | | | |
| CN | 115105198 | A | 27 September 2022 | None | | | |
| CN | 109330667 | A | 15 February 2019 | None | | | |
| US | 2010228534 | A1 | 09 September 2010 | JP | 2010520006 | A | 10 June 2010 |
| | | | | EP | 2114255 | A2 | 11 November 2009 |
| | | | | BRPI | 0807295 | A2 | 06 May 2014 |
| | | | | WO | 2008107874 | A2 | 12 September 2008 |

Form PCT/ISA/210 (patent family annex) (July 2022)